(19) **Europäisches Patentamt / European Patent Office / Office européen des brevets**

(11) **EP 2 022 395 B1**

(12) **FASCICULE DE BREVET EUROPEEN**

(45) Date de publication et mention
de la délivrance du brevet:
**01.09.2010 Bulletin 2010/35**

(51) Int Cl.:
**A61B 5/0215** *(2006.01)*   **A61B 5/03** *(2006.01)*

(21) Numéro de dépôt: **08104806.8**

(22) Date de dépôt: **21.07.2008**

(54) **Capteur de pression auto-étalonnable**

Selbsteichender Drucksensor

Self-calibrating pressure sensor

(84) Etats contractants désignés:
**AT BE BG CH CY CZ DE DK EE ES FI FR GB GR
HR HU IE IS IT LI LT LU LV MC MT NL NO PL PT
RO SE SI SK TR**

(30) Priorité: **31.07.2007 FR 0705573**

(43) Date de publication de la demande:
**11.02.2009 Bulletin 2009/07**

(73) Titulaires:
• **Captomed EURL**
**31670 Labege (FR)**
• **Centre National de la Recherche Scientifique
(CNRS)
75016 Paris (FR)**

(72) Inventeurs:
• **Pons, Patrick
31750 Escalquens (FR)**
• **Montoriol, Pierre
31290 Monremont (FR)**
• **Yameogo, Pierre
31100 Toulouse (FR)**

(74) Mandataire: **Ravina, Bernard**
**Ravina S.A.
8, rue des Briquetiers
Z.A. de Font Grasse
31703 Blagnac cedex (FR)**

(56) Documents cités:
**WO-A-2006/073770      US-A- 5 437 284
US-A1- 2006 107 749      US-B1- 6 475 170**

## Description

La présente invention appartient au domaine des capteurs de pression et plus particulièrement des capteurs destinés au contrôle de la pression intracorporelle d'un patient durant une période de surveillance médicale, notamment au cours d'une intervention chirurgicale.

Elle a pour objet un dispositif auto-étalonnable de mesure de la pression intracorporelle, associant un capteur à transducteur piézoélectrique réagissant sous l'effet d'une pression et un actionneur permettant de provoquer, par une polarisation d'amplitude déterminée, une déformation calibrée au niveau du capteur.

Les mesures de pression sont couramment utilisées pour juger de l'état des fonctions physiologiques vitales, ou de la gravité d'un traumatisme, d'une pathologie qui engendre la compression anormalement élevée d'un organe. Ces mesures de pression, en particulier la pression artérielle et la pression intracrânienne, sont effectuées de façon journalière dans les services d'urgences notamment en réanimation.

La mesure de pression artérielle est nécessaire dans de nombreuses situations telles que les états de choc ne répondant pas à la première heure de prise en charge médicale (choc septique, choc cardiogénique), les états de mal convulsif réfractaire ou le syndrome de détresse respiratoire aigüe. La prise en charge postopératoire de multiples situations comme la greffe rénale, la chirurgie orthopédique lourde, la chirurgie digestive lourde, où l'intervention prolongée requiert de nombreux remplissages vasculaires qui peuvent conduire à une gestion hémodynamique postopératoire houleuse, nécessitent également un suivi étroit de la pression artérielle.

La mesure de la pression intracrânienne est indiquée en neuro-traumatologie dans les cas de traumatismes crâniens graves et également dans certains cas d'oedèmes cérébraux relationnels, d'hémorragies méningées ou de tumeurs cérébrales fortement expansives. Tous ces processus pathologiques ont en commun de produire, par effet de masse, une hyper tension intracrânienne, puisque la boîte crânienne est inextensible chez l'adulte. Une augmentation de la pression intracrânienne produit une diminution de la pression de perfusion cérébrale qui peut être responsable d'une baisse du débit sanguin cérébral et créer une situation de menace énergétique pouvant conduire à l'ischémie, c'est à dire la mort du tissu cérébral.

On comprend que le contrôle et la régulation de la pression intracorporelle puisse revêtir une importance de premier ordre, souvent vitale, dans des contextes particulièrement difficiles pour les praticiens. Il a donc été recherché de longue date des moyens de mesurer aussi bien la pression intracrânienne que la pression artérielle, qui soient adaptés au travail en salle d'opération ou en environnement médical, requérant des outils peu encombrants et maniables aptes à fournir des informations fiables en continu et dans des conditions de stricte asepsie.

De façon classique, la mesure de pression artérielle est réalisée à l'aide d'un capteur qu'on introduit dans l'artère radiale ou fémorale par l'intermédiaire d'un cathéter. Le principe du capteur est celui d'une membrane couplée à des jauges à résistances placées dans un pont de Wheatstone. Les variations de résistance dues à la compression ou à l'extension des jauges reliées à la membrane sont transformées en un signal électrique proportionnel à la pression artérielle. Les résistances peuvent être remplacées par un système induisant une variation d'un couplage électromagnétique en fonction des déplacements de la membrane. Le capteur peut être externe, ce qui présente différents inconvénients liés à l'éloignement du capteur, en relation notamment avec des effets d'amortissement et de déformation du signal de pression et une limitation de la réponse en fréquence.

Aussi emploie-t-on de plus en plus fréquemment, en particulier en salle d'opération, des capteurs de pression intégrés à l'extrémité implantée d'un cathéter. Depuis 1980, on utilise communément des capteurs basés sur des technologies à fibres optiques ou à transducteurs piézoélectriques (en particulier à jauges piézorésistives) en silicium. Les composants à jauges sont encapsulés à l'extrémité d'un cathéter en nylon d'un diamètre hors tout, variant de 1,17 mm à 2,67 mm pour les modèles commercialisés. Grâce à la miniaturisation de la sonde de mesure, les risques de thrombose lors de la prise de la pression artérielle ont pu être limités, et le traumatisme lié à l'implantation intracrânienne du capteur a été minimisé. Ces dispositifs ont ainsi permis ces dernières années, de mesurer la pression artérielle et la pression intracrânienne à un coût moindre, de façon routinière, avec une bien meilleure précision des mesures, un caractère invasif réduit et des risques septiques mieux maîtrisés.

US 2006/107749 décrit un capteur selon la préambule de la revendication 1.

Il n'en demeure pas moins que ces techniques présentent encore des inconvénients et appellent des innovations. Un problème majeur des capteurs à jauges en silicium et à fortiori des capteurs à fibres optiques actuels, est leur propension à dériver, que ce soit en conditions de stockage ou d'utilisation. Des valeurs de 2 à 5 mm Hg par cycles de 24 heures sont couramment observées. Il est donc indispensable de réaliser un réétalonnage à intervalles de temps réguliers. Or à l'heure actuelle, l'étalonnage ne peut être réalisé que de manière extracorporelle, ce qui accroît très sérieusement les risques infectieux pour les patients.

En effet, l'étalonnage des capteurs de pression est en général réalisé en appliquant une pression connue sur la membrane du capteur et en vérifiant que la grandeur électrique délivrée par celui-ci est en accord avec la contrainte exercée. Dans la pratique, les capteurs utilisés dans le milieu médical délivrant une valeur de pression égale à la différence entre la pression mesurée et la pression atmosphérique, un capteur est étalonné à la pression atmosphérique : s'il est correctement calibré, la pression mesurée doit être nulle quand il est tenu à l'air libre ou plongé dans un faible volume d'eau stérile.

Or, la manipulation d'un instrument placé dans l'organisme, puis retiré et réintroduit, est par nature délicate et source de complications infectieuses dont les conséquences peuvent s'avérer extrêmement sérieuses. Pour la mesure de la pression intracrânienne notamment, l'émergence du lien physique, électrique ou optique, avec le milieu extérieur au niveau du scalp est toujours un point critique. Il est une possible porte d'entrée septique vers le milieu intradural dont la vulnérabilité infectieuse est totale. La mise en place répétée périodiquement d'un appareillage multiplie donc significativement les risques infectieux. Ainsi, du fait d'un risque septique élevé, il est préférable qu'un capteur posé ne soit plus retiré pour subir des réétalonnages périodiques.

Le but de la présente invention est d'apporter une réponse aux inconvénients précités en proposant un capteur de pression dont la dérive dans le temps peut être aisément contrôlée et corrigée. Un autre but de l'invention est de fournir un capteur de pression utilisable pour des mesures intracorporelles, pouvant être étalonné *in situ*. Ces objectifs impliquent miniaturisation, fiabilité, sensibilité du dispositif.

La présente invention répond à ces objectifs en proposant un dispositif auto-étalonnable de mesure de la pression intracorporelle, comprenant :

- un capteur de pression comportant i) un corps d'épreuve dont un segment de moindre épaisseur constitue une membrane susceptible de se déformer sous l'effet de la pression du milieu, ladite membrane étant dotée d'au moins un transducteur piézoélectrique apte à traduire une contrainte mécanique en signal électrique, et ii) un contact de polarisation,
- un actionneur connecté à une source de tension, comportant une base non déformable, une face au moins dudit actionneur étant polarisable,

ledit capteur de pression étant placé au-dessus dudit actionneur de manière à ce que la membrane soit à l'aplomb de la face polarisable.

Dans le dispositif objet de l'invention sont donc associés un capteur et un actionneur. Par soucis de simplicité, on considère dans la présente description que le capteur est placé au-dessus de l'actionneur, bien qu'il s'agisse ici uniquement d'une position relative entre ces deux éléments qui peuvent tout à fait fonctionner quelle que soit leur position dans l'espace. On rappelle qu'un capteur est un organe qui convertit une grandeur physique en grandeur électrique. Un actionneur est un organe qui convertit une grandeur électrique en une grandeur physique (non-électrique). Dans le cas présent, l'actionneur est apte à transformer une tension électrique en une force électrostatique provoquant une déformation. Celle-ci est subie par le capteur qui la transforme en signal électrique.

La première partie du dispositif selon l'invention est un capteur de pression, qui peut être du type des capteurs de pression à transducteurs piézoélectriques déjà connus en eux-mêmes. Le fonctionnement de ces capteurs est basé sur la réaction de transducteurs piézorésistifs (appelés aussi jauges de contrainte) insérés dans une membrane se déformant sous l'effet d'une pression, dont la variation de résistance traduit leur propre déformation et donc la déformation du corps d'épreuve sur lequel ils sont installés.

Le capteur est donc principalement constitué d'un corps d'épreuve chargé de retranscrire la pression qui lui est appliquée (par déformation de sa surface due à une pression externe à mesurer ou à la force électrostatique générée à l'occasion du calibrage), sur lequel une fine membrane est ménagée. On peut le réaliser par exemple grâce aux techniques de micro-usinage employées en microélectronique. Des transducteurs piézoélectriques, également réalisés grâce à des techniques connues de microélectronique, peuvent ensuite être disposés dans les zones les plus adéquates. Les variations de leurs caractéristiques électriques retranscrivent tout mouvement ou déformation de la membrane.

Le capteur comporte en outre un contact de polarisation, de sorte que lorsque l'actionneur est sous tension, une force électrostatique peut s'exercer sur la membrane sensible.

Le capteur est associé à un actionneur comportant une base non déformable. Ce dernier est connecté à une source de tension et une de ses faces au moins est polarisable. Il est chargé de provoquer une déformation connue de la membrane par polarisation électrique. Pour cela, le capteur de pression est placé vis-à-vis dudit actionneur de manière à ce que la membrane du capteur soit à l'aplomb de la face polarisable de l'actionneur.

En effet, une force électrostatique peut être générée quand deux surfaces conductrices parallèles sont mises en regard et qu'une différence de potentiel leur est appliquée. La force qui attire ou repousse les électrodes (en fonction du signe de la tension qui leur est appliquée) peut être approximée par l'équation suivante :

$$F = \frac{\varepsilon \times S}{2d^2} \times U^2,$$

dans laquelle $\varepsilon$ est la permittivité du milieu, $S$ représente la surface des électrodes mises en regard, $d$ est la distance entre les électrodes et $U$ est la tension appliquée aux électrodes.

Dans la présente application, la membrane du capteur de pression joue le rôle d'une première électrode, la face polarisable de l'actionneur constituant la deuxième électrode. En positionnant une électrode conductrice non déformable à l'aplomb de la membrane sensible, on crée un actionneur électrostatique qui permet d'appliquer une force sur la membrane et ainsi de la déformer. Cette déformation est transformée par les transducteurs piézoélectriques en signaux électriques. Pour des valeurs de *S*, *d* et ε prédéterminées, la tension *U* appliquée entre l'électrode et la membrane étant connue, la déflexion subie par la membrane est connue. Cette déflexion étant équivalente à celle obtenue par l'application d'une pression donnée, le signal électrique fourni par le capteur est proportionnel à cette pression, qui constitue ainsi une pression de calibration à partir de laquelle les valeurs de la pression intracorporelle mesurées par le capteur peuvent être rectifiées.

Grâce à ce dispositif, il est possible d'appliquer à volonté à la membrane du capteur une contrainte connue, revenant à appliquer une pression connue au capteur, par l'intermédiaire de l'actionneur associé au capteur. Le capteur peut ainsi être étalonné aussi souvent que de besoin sans qu'il soit nécessaire de le retirer du milieu dans lequel il est placé, par une mise sous tension périodique du dispositif selon l'invention.

Selon une caractéristique avantageuse du dispositif objet de l'invention, le capteur est maintenu au-dessus de l'actionneur par une liaison rigide, l'espace compris entre la membrane et la face polarisable constituant une cavité de dimension déterminée : la distance d entre les électrodes est ainsi fixée. On dispose alors d'un dispositif monolithique à actionneur embarqué, que sa taille réduite rend apte à être inséré dans un organisme vivant par l'intermédiaire d'un cathéter ou autre.

Selon une autre caractéristique du dispositif selon l'invention, l'actionneur comprend des moyens d'isolation séparant la face polarisable du corps d'épreuve. L'actionneur peut être muni d'un élément isolant le séparant de la face polarisable et le corps d'épreuve, ou encore être lui-même réalisé selon une structure et dans un matériau assurant la fonction d'isolation, comme il apparaîtra par la suite. La liaison rigide notamment, ne doit pas autoriser le passage du courant électrique.

Dans une variante d'exécution du dispositif selon l'invention, l'actionneur comporte, sur un substrat isolant non déformable, une électrode métallique connectée à une source de tension. Cette électrode métallique constitue une face polarisable de l'actionneur, placée à l'aplomb la membrane et sert d'actuateur électrostatique permettant de générer une déformation connue de la membrane. La fabrication de l'électrode peut être réalisée par différents procédés, par exemple par un des procédés déjà utilisés pour la réalisation des capteurs de pression capacitifs. En pratique, l'électrode métallique est constituée d'une couche de métal recouvrant le substrat au moins sur la surface se trouvant à l'aplomb de la membrane.

Le substrat isolant peut être quant à lui avantageusement constitué d'un matériau choisi parmi le verre, le verre borosilicaté, la vitrocéramique, le silicium isolant. Tout autre matériau isolant possédant les propriétés requises de rigidité peut être employé.

Selon une variante alternative de mise en oeuvre de l'invention, l'actionneur comporte un substrat conducteur non déformable connecté à une source de tension et séparé du corps d'épreuve par au moins un élément isolant.

Dans ce cas, le substrat conducteur est de préférence en silicium conducteur tandis que ledit au moins un élément isolant est en oxyde de silicium.

Quel que soit le mode de réalisation de l'actionneur associé au capteur de pression, le corps d'épreuve et la membrane dudit capteur sont réalisés en un matériau conducteur, de préférence en silicium. On peut utiliser pour leur fabrication, par exemple, un procédé de photo-lithogravure du silicium, la gravure étant réalisée par voie chimique ou grâce à un plasma, selon des modalités connues de l'homme de l'art.

De manière avantageuse selon l'invention, ledit capteur de pression comprend au moins deux, et de préférence quatre transducteurs piézoélectriques, placés dans les zones de plus grande déformation de la membrane.

Selon une caractéristique avantageuse également, ledit au moins un transducteur piézoélectrique est choisi parmi les jauges piézorésistives, les diodes à jonction PN, les transistors bipolaires ou les transistors à effet de champ. De nos jours, les résistances restent l'élément piézoélectrique le plus couramment utilisé à la surface des membranes, mais tout composant peut être utilisé pour traduire un comportement mécanique de la membrane en comportement électrique. Plusieurs jauges ayant des caractéristiques piézorésistives identiques ou différentes peuvent être fabriquées sur un même substrat et agencées selon différentes configurations (montage en simple pont, pont de Wheatstone, etc..), améliorant leurs performances et diminuant l'impact des grandeurs d'influence (la température principalement).

Ces jauges peuvent être obtenues par différents procédés. Dans un mode de réalisation préféré du dispositif selon la présente invention, les jauges piézorésistives sont en silicium dopé intégré dans ladite membrane en silicium. Dans ce cas, les jauges piézorésistives ne sont pas fixées sur le corps d'épreuve, mais sont intégrées au matériau (ici le silicium), c'est-à-dire qu'elles sont parties constituantes de la membrane. En effet, on agit au niveau atomique, par dopage ciblé des zones de la membrane où les déformations sont maximales. Les zones dopées retranscrivent la pression appliquée à la membrane par une variation de leurs caractéristiques électriques. Ces dopages de la surface du matériau peuvent être réalisés par des techniques utilisées en microélectronique, telles que la diffusion atomique ou l'implantation ionique.

Ainsi, dans sa forme de réalisation préférée, la mise en oeuvre de l'invention peut avantageusement s'appuyer sur les technologies connues du silicium à transducteurs piézorésistifs et sur les méthodes de fabrication de membranes sensibles mises au point à ces fins.

Le dispositif selon l'invention peut être un capteur relatif. Dans ce cas, la cavité est prolongée par un canal ouvert sur l'atmosphère.

Le dispositif selon l'invention peut aussi être un capteur absolu. Dans ce cas, au contraire, la cavité est hermétiquement fermée par soudure sous vide du capteur avec l'actionneur (celui-ci est alors constitué d'un substrat conducteur).

Selon une caractéristique intéressante du dispositif objet de la présente invention, celui-ci peut comprendre en outre un capteur de température. Celui-ci est peut être réalisé par une des techniques à la disposition de l'homme de l'art.

Par exemple, il pourra être constitué d'une thermistance constituée, soit d'un métal type nickel ou platine déposé par pulvérisation (selon un principe largement utilisé dans les capteurs de température type Pt100 ou Pt1000), soit d'un semi-conducteur dopé, du silicium ou du polysilicium par exemple. Ce type de capteur est le plus souvent utilisé seul, en composant discret.

Il pourra de manière alternative être constitué d'une sonde de température en silicium à saut de bande, comprenant des diodes ou des transistors traversés par deux courants différents dont on mesure la différence entre les tensions de seuil (cas des diodes) ou les tensions Emetteur-Base (cas des transistors). La tension ainsi mesurée est directement proportionnelle à la température. Ce type de capteurs, qui tire son nom de la référence de tension à saut de bande utilisée pour générer les courants traversant les diodes ou les transistors, est largement utilisé dans l'industrie microélectronique.

Comme déjà indiqué, les transducteurs piézoélectriques du dispositif selon l'invention retranscrivent le déplacement de la membrane induit par le champ électrique lorsque l'actionneur est sous tension, sous la forme d'un signal électrique. Celui-ci, une fois traité et analysé, donnera des informations sur la dérive éventuelle du capteur. Pour cela, le corps d'épreuve comprend bien entendu des moyens de connexion électrique des transducteurs piézoélectriques et du contact de polarisation.

Selon une autre caractéristique particulière de l'invention, le dispositif selon l'invention est avantageusement relié à une unité centrale comprenant des moyens de traitement du signal électrique généré par le capteur de pression, et éventuellement par le capteur de température. Le signal électrique généré par les transducteurs piézoélectriques peut donc être enregistré, traité et comparé à des valeurs préalablement acquises dans les mêmes conditions d'actuation électrostatique, pour évaluer et éventuellement corriger une possible dérive des transducteurs piézoélectriques, en un mot pour étalonner le capteur, aussi souvent que nécessaire. Cette correction est avantageusement réalisée à l'aide d'un programme d'ordinateur adéquat.

Le processus d'étalonnage périodique et d'ajustement de la valeur de la pression mesurée peut ainsi être réalisé de manière automatisée, par simple actionnement du seul dispositif de mesure objet de la présente invention, ce qui autorise à qualifier le présent dispositif d'auto-étalonnable. Cet étalonnage permet de corriger aisément, instantanément et sans risque sanitaire, la dérive dans le temps des valeurs de la pression intracorporelle mesurée, principale faiblesse des capteurs de pression actuellement utilisés.

Pour sa mise en oeuvre commode et sûre notamment dans un usage médical, le dispositif peut être fixé à l'extrémité d'un cathéter et encapsulé dans une gaine de résine comportant une fenêtre au niveau de la membrane.

Finalement, est également objet de la présente invention un cathéter à usage médical dont l'extrémité destinée à être implantée dans le corps d'un patient comporte un dispositif auto-étalonnable de mesure de la pression intracorporelle tel que précédemment décrit.

La présente invention sera mieux comprise, et des détails en relevant apparaîtront, à la lumière de la description qui va être faite de différentes variantes de réalisation, en relation avec les figures des planches annexées, dans lesquelles :

La figure 1 est une représentation schématique en coupe d'un dispositif selon l'invention, associant un capteur relatif et un actionneur doté d'une électrode métallique sur un substrat isolant.

La figure 2 est une vue schématique en coupe d'un dispositif selon l'invention, associant un capteur absolu et un actionneur comportant un substrat conducteur.

La figure 3 est une vue de dessus schématique des dispositifs représentés aux figures 1 et 2, faisant apparaître uniquement les composants et les circuits électriques du capteur.

**EXEMPLE 1 : Capteur relatif associé à un actionneur à électrode métallique**

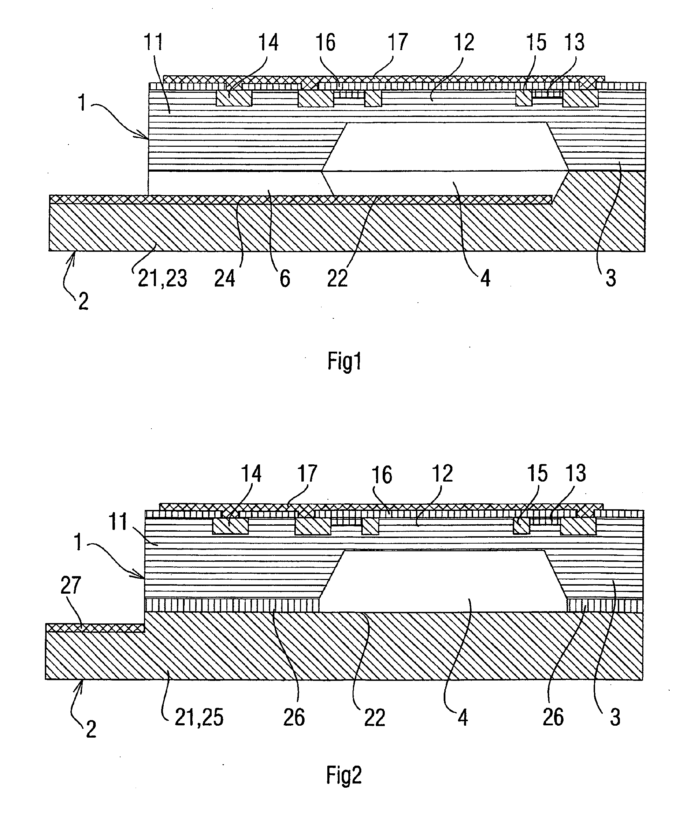

En se référant à la figure 1, le dispositif selon cet exemple comprend le capteur de pression 1, qui comporte d'une part le corps d'épreuve 11 et d'autre part le contact de polarisation 14. Le corps d'épreuve 11 a un segment de moindre épaisseur qui constitue la membrane 12, susceptible de se déformer sous l'effet de la pression du milieu ou d'une force électrostatique. Le corps d'épreuve 11 du capteur 1, y compris la membrane 12, est réalisé en silicium conducteur.

Ladite membrane est dotée de quatre transducteurs piézoélectriques 13, aptes à traduire une contrainte mécanique en signal électrique. Les transducteurs 13 choisis sont des jauges piézorésistives placées dans les zones de plus grande déformation de la membrane 12. Quatre jauges montées en pont de Wheatstone sont utilisées. Elles sont intégrées à la membrane de silicium 12 par dopage ciblé des zones où les déformations sont maximales, c'est-à-dire sur chacun des côtés à la périphérie de la membrane 12. Par exemple, la membrane 12 s'inscrit dans un parallélogramme comportant un transducteur 13 sur chaque côté. Des interconnexions 15 fortement dopées P++ sont ménagées de part et d'autre des jauges 13. Le contact de polarisation 14 est quant à lui assuré grâce à un fort dopage N++. Les variations des caractéristiques électriques des jauges 13 retranscrivent tout mouvement ou déformation de la membrane, provoqué par la pression à mesurer ou par le champ électrique induit par la polarisation du montage.

Le dispositif comprend aussi l'actionneur 2 connecté à une source de tension, comportant la base 21 non déformable, avec la face polarisable 22. L'actionneur 2 est constituée du substrat 23 isolant non déformable et de l'électrode métallique 24 connectée à une source de tension. Cette électrode métallique constitue une face polarisable de l'actionneur 2, placée à l'aplomb la membrane 12. Elle est constituée d'une couche de métal Ti-Au ou Al recouvrant le substrat isolant 23 sur une partie de sa surface, et particulièrement sur la surface se trouvant à l'aplomb de la membrane 12. Le substrat isolant 23 est réalisé en verre borosilicaté (plus connu sous le nom commercial de Pyrex™). C'est ce matériau qui assure la fonction d'isolation, en séparant la face polarisable 22 et le corps d'épreuve 11.

Le capteur de pression 1 est placé au-dessus de l'actionneur 2 (selon la représentation ici adoptée) de manière à ce que la membrane 12 soit à l'aplomb de la face polarisable 22. Il est maintenu à celui-ci par la liaison rigide 3, l'espace compris entre la membrane 12 et la face polarisable 22 constituant la cavité 4 de dimension déterminée : la distance d entre les électrodes est ainsi comprise entre 100 nm et 300 nm. La liaison 3 est assurée par collage des extensions respectives en bordure du capteur 1 et le l'actionneur 2, par exemple par la technique de soudure anodique. Elle n'autorise pas le passage du courant électrique. La cavité 4 est prolongée par le canal 6 ouvert sur l'atmosphère. Le capteur est donc du type capteur relatif, fonctionnant par référence à la pression atmosphérique.

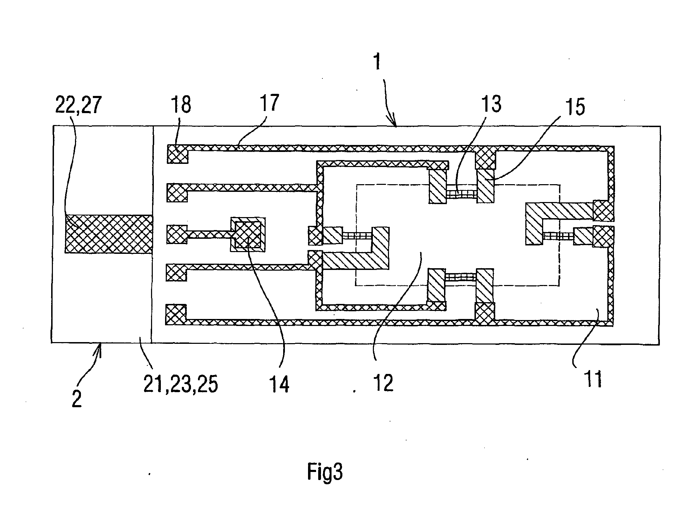

Pour son fonctionnement, le corps d'épreuve 11 comprend des moyens de connexion électrique des jauges piézorésistives 13 et du contact de polarisation 14. L'agencement des pistes métalliques 17 et des plots de connexion 18 est représenté sur la figure 3. Les pistes métalliques 17 sont isolées de la membrane 12 par la couche isolante 16 (omise sur la figure 3).

La fabrication du dispositif ici décrit recourt aux technologies connues appliquées au silicium à transducteurs piézoélectriques et aux méthodes de fabrication de membranes sensibles mises au point à ces fins, que l'homme du métier sait choisir et mettre en oeuvre.

Le dispositif auto-étalonnable de mesure de la pression intracorporelle peut être installé à l'extrémité d'un cathéter. Pour cela, il peut être fixé à l'extrémité d'un ruban souple, par exemple en polyimide, supportant également les câbles de connexion, et introduit dans le cathéter jusqu'à émerger à son extrémité. Il est alors encapsulé dans une gaine comportant une fenêtre au niveau de la membrane. La capsule peut-être constituée de manière connue par un demi cylindre dans lequel on glisse le capteur auto-étalonnable, puis qu'on ferme par micro-dépose d'une colle. Le dispositif ainsi encapsulé à l'extrémité d'un cathéter a un diamètre hors tout, de l'ordre de 1 mm à 1,2 mm convenant à une introduction dans l'organisme d'un patient.

Pour la fabrication de ce dispositif, on pourra procéder selon les étapes principales suivantes :

- Préparation d'un substrat de Pyrex™, en vue de la réalisation d'une cavité avec canal de sortie, permettant la connexion électrique de l'électrode métallique ;
- Dépôt d'une électrode de métal sur le substrat ;
- Réalisation d'un corps d'épreuve en silicium avec membrane ;
- Implantation des jauges piézorésistives sur le corps d'épreuve ;
- Implantation des interconnexions P++ pour les jauges et N++ pour la polarisation de la membrane ;
- Métallisation pour l'interconnexion des jauges en pont de Wheatstone et du contact de polarisation de la membrane
- Soudure de la plaque actionneur et de la plaque capteur pour créer la cavité avec deux surfaces polarisables en vis-à-vis ;
- Découpage des composants.

**EXEMPLE 2 : Capteur absolu associé à un actionneur en silicium conducteur**

En se référant à la figure 2, le dispositif selon cet exemple comprend le capteur de pression 1, qui comporte d'une part le corps d'épreuve 11 et d'autre part le contact de polarisation 14. Le corps d'épreuve 11 a un segment de moindre épaisseur qui constitue la membrane 12, susceptible de se déformer sous l'effet de la pression du milieu. Le corps d'épreuve 11 du capteur 1, y compris la membrane 12, est réalisé en silicium conducteur.

Ladite membrane est dotée de quatre transducteurs piézoélectriques 13, aptes à traduire une contrainte mécanique en signal électrique. Les transducteurs 13 choisis sont des jauges piézorésistives placées dans les zones de plus grande déformation de la membrane 12. Quatre jauges sont intégrées à la membrane 12 de silicium par dopage ciblé des zones où les déformations sont maximales, c'est-à-dire sur chacun des côtés à la périphérie de la membrane 12. Des interconnexions P++ 15 sont ménagées de part et d'autre des jauges 13. Des interconnexions N++ sont prévues pour le contact de polarisation 14.

Le dispositif comprend aussi l'actionneur 2 connecté à une source de tension, comportant la base 21 non déformable, avec la face polarisable 22. L'actionneur 2 est constituée du substrat conducteur 25 non déformable connecté à une source de tension par un dépôt métallique 27 et séparé du corps d'épreuve 11 par l'élément isolant 26. Le substrat 25 est en silicium conducteur tandis que l'élément isolant 26 est en oxyde de silicium. C'est ce dernier matériau qui assure la fonction d'isolation en séparant la face polarisable et le corps d'épreuve. La surface du substrat conducteur constitue une face polarisable de l'actionneur 2 placée à l'aplomb la membrane 12.

Le capteur de pression 1 est placé au-dessus de l'actionneur 2 (selon la représentation ici adoptée) de manière à ce que la membrane 12 soit à l'aplomb de la face polarisable 22. Il est maintenu à celui-ci par la liaison rigide 3 l'espace compris entre la membrane 12 et la face polarisable 22 constituant la cavité 4 : la distance d entre les électrodes est ainsi fixée à environ 1 mm. La liaison 3 est assurée par collage des extensions en oxyde de silicium, en bordure du dispositif du capteur 1 et le l'actionneur 2, par exemple par soudure par fusion. Elle n'autorise pas le passage du courant électrique. Dans ce cas, la cavité est scellée avec une soudure réalisée sous vide. Le capteur est donc du type capteur absolu.

Comme pour l'exemple précédent, le corps d'épreuve 11 comprend des moyens de connexion électrique des jauges piézorésistives 13 montées en pont de Wheatstone et du contact de polarisation 14. L'agencement des pistes métalliques 17 et des plots de connexion 18 est représenté sur la figure 3. Les pistes métalliques 17 sont isolées de la membrane 12 par la couche isolante 16 (omise sur la figure 3). Le dispositif peut être installé à l'extrémité d'un cathéter et encapsulé, comme déjà décrit.

On pourra fabriquer ce dispositif selon les étapes principales suivantes :

- Réalisation d'un corps d'épreuve en silicium avec membrane
- Croissance d'une couche d'oxyde isolant pour préparer la liaison avec l'actionneur ;
- Implantation des jauges piézorésistives sur le corps d'épreuve ;
- Implantation des interconnexions P++ pour les jauges et N++ pour la polarisation de la membrane ;
- Métallisation pour l'interconnexion des jauges en pont de Wheatstone et du contact de polarisation de la membrane ;
- Préparation d'une plaque de silicium conducteur (actionneur) ;
- Soudure de la plaque actionneur et de la plaque capteur pour créer une cavité avec deux surfaces polarisables en vis-à-vis ;
- Métallisation d'une partie de la plaque actionneur pour permettre la connexion électrique de la plaque et sa polarisation ;
- Découpage des composants ;
- Scellement de la cavité sous vide lors de la soudure.

**Revendications**

1. Dispositif de mesure de la pression intracorporelle, comprenant :

un capteur de pression (1) comportant un corps d'épreuve (11) dont un segment de moindre épaisseur constitue une membrane (12) susceptible de se déformer sous l'effet de la pression du milieu, ladite membrane étant dotée d'au moins un transducteur piézoélectrique (13) apte à traduire une contrainte mécanique en signal électrique,
et **caractérisé en ce que**

- le capteur comporte aussi un contact de polarisation (14),
- le dispositif est auto-étalonnable et il comporte un actionneur (2) connecté à une source de tension, comportant une base (21) non déformable, une face (22) au moins dudit actionneur étant polarisable,

ledit capteur de pression étant placé au-dessus dudit actionneur de manière à ce que la membrane (12) soit à l'aplomb de la face polarisable (22).

2. Dispositif selon la revendication 1, **caractérisé en ce que** le capteur (1) est maintenu au-dessus de l'actionneur (2) par une liaison rigide (3), l'espace compris entre la membrane (12) et la face polarisable (22) constituant une cavité (4) de dimension déterminée.

3. Dispositif selon la revendication 1 ou 2, **caractérisé en ce que** l'actionneur (2) comprend des moyens d'isolation séparant la face polarisable (22) et le corps d'épreuve (11).

4. Dispositif selon l'une des revendications précédentes, **caractérisé en ce que** l'actionneur (2) comporte, sur un substrat isolant (23) non déformable, une électrode (24) métallique connectée à une source de tension.

5. Dispositif selon la revendication 4, **caractérisé en ce que** le substrat isolant (23) est constitué d'un matériau choisi parmi le verre, le verre borosilicaté, la vitrocéramique, le silicium isolant.

6. Dispositif selon l'une des revendications 1 à 3, **caractérisé en ce que** l'actionneur (2) comporte un substrat conducteur (25) non déformable connecté à une source de tension et séparé du corps d'épreuve par au moins un élément isolant (26).

7. Dispositif selon la revendication précédente, **carac-**

**térisé en ce que** le substrat conducteur (25) est en silicium conducteur et ledit au moins un élément isolant (26) est en oxyde de silicium.

8. Dispositif selon l'une quelconque des revendications précédentes, **caractérisé en ce que** le corps d'épreuve (11) et la membrane (12) dudit capteur de pression sont en silicium conducteur.

9. Dispositif selon l'une des revendications précédentes, **caractérisé en ce que** ledit capteur de pression comprend au moins deux, et de préférence quatre transducteurs piézoélectriques (13) placés dans les zones de plus grande déformation de la membrane (12).

10. Dispositif selon l'une des revendications précédentes, **caractérisé en ce que** ledit au moins un transducteur piézoélectrique est choisi parmi les jauges piézorésistives, les diodes à jonction PN, les transistors bipolaires ou les transistors à effet de champ.

11. Dispositif selon l'une des revendications précédentes, **caractérisé en ce que** lesdites jauges piézorésistives sont en silicium dopé intégré dans ladite membrane en silicium.

12. Dispositif selon l'une des revendications précédentes, **caractérisé en ce que** la cavité (4) est prolongée par un canal (6) ouvert sur l'atmosphère.

13. Dispositif selon l'une des revendications 1 à 12, **caractérisé en ce que** la cavité (4) est hermétiquement fermée et vide d'air.

14. Dispositif selon l'une quelconque des revendications précédentes, **caractérisé en ce qu'**il comprend en outre un capteur de température.

15. Dispositif selon l'une quelconque des revendications précédentes, **caractérisé en ce qu'**il est relié à une unité centrale comprenant des moyens de traitement du signal électrique généré par le capteur de pression (1), et éventuellement par le capteur de température.

16. Dispositif selon l'une quelconque des revendications précédentes, **caractérisé en ce qu'**il est fixé à l'extrémité d'un cathéter et encapsulé dans une gaine de résine comportant une fenêtre au niveau de la membrane (12).

17. Cathéter à usage médical **caractérisé en ce que** l'extrémité destinée à être implantée dans le corps d'un patient comporte un dispositif auto-étalonnable de mesure de la pression intracorporelle selon l'une des revendications précédentes.

**Claims**

1. Device for measuring intracorporeal pressure comprising:

   a pressure sensor (1) comprising a test body (11) of which a segment of smaller thickness constitutes a membrane (12) that can deform under the action of the pressure of the medium, said membrane being equipped with at least one piezoelectric transducer (13) that can translate a mechanical stress into an electrical signal, and **characterized in that**

   - the sensor further comprises a polarization contact (14),
   - the device is self-calibrating and comprises an actuator (2) that is connected to a voltage source, comprising a non-deformable base (21), at least one surface (22) of said actuator being polarizable,

   said pressure sensor being placed above said actuator so that the membrane (12) is straight above the polarizable surface (22).

2. Device according to claim 1, **characterized in that** the sensor (1) is kept above the actuator (2) by a rigid connection (3), the space that is encompassed between the membrane (12) and the polarizable surface (22) constituting a cavity (4) of specified size.

3. Device according to claim 1 or 2, **characterized in that** the actuator (2) comprises insulating means separating the polarizable surface (22) and the test body (11).

4. Device according to one of the preceding claims, **characterized in that** the actuator (2) comprises, on a non-deformable insulating substrate (23), a metal electrode (24) that is connected to a voltage source.

5. Device according to claim 4, **characterized in that** the insulating substrate (23) consists of a material selected from among glass, borosilicate glass, vitreous ceramic, and insulating silicon.

6. Device according to one of claims 1 to 3, **characterized in that** the actuator (2) comprises a non-deformable conductive substrate (25) that is connected to a voltage source and separated from the test body by at least one insulating element (26).

7. Device according to the preceding claim, **characterized in that** the conductive substrate (25) is made of conductive silicon, and said at least one insulating element (26) is made of silicon oxide.

8. Device according to any of the preceding claims, **characterized in that** the test body (11) and the membrane (12) of said pressure sensor are made of conductive silicon.

9. Device according to one of the preceding claims, **characterized in that** said pressure sensor comprises at least two, and preferably four, piezoelectric transducers (13) that are placed in the zones of greater deformation of the membrane (12).

10. Device according to one of the preceding claims, **characterized in that** said at least one piezoelectric transducer is selected from among the piezoresistive gauges, the pn-junction diodes, the bipolar transistors, or the field-effect transistors.

11. Device according to one of the preceding claims, **characterized in that** said piezoresistive gauges are made of doped silicon that is integrated into said silicon membrane.

12. Device according to one of the preceding claims, **characterized in that** the cavity (4) is extended by an open channel (6) into the atmosphere.

13. Device according to one of claims 1 to 12, **characterized in that** the cavity (4) is hermetically sealed and devoid of air.

14. Device according to any of the preceding claims, **characterized in that** it also comprises a temperature sensor.

15. Device according to any of the preceding claims, **characterized in that** it is connected to a central unit that comprises electric signal processing means generated by the pressure sensor (1) and optionally by the temperature sensor.

16. Device according to any of the preceding claims, **characterized in that** it is attached at the end of a catheter and encapsulated in a resin sheath that comprises a window at the level of the membrane (12).

17. Catheter for medical use, **characterized in that** the end that is designed to be implanted in a patient's body comprises a self-calibrating device for measuring the intracorporeal pressure according to one of the preceding claims.

**Patentansprüche**

1. Vorrichtung zur Messung des Körperinnendrucks, umfassend:

einen Drucksensor (1), der einen Prüfkörper (11) aufweist, von welchem ein Abschnitt geringerer Dicke eine Membran (12) bildet, die sich unter Einwirkung des Umgebungsdrucks verformen kann, wobei die Membran mit mindestens einem piezoelektrischen Wandler (13) versehen ist, der dazu befähigt ist, eine mechanische Belastung in ein elektrisches Signal umzuwandeln, und **dadurch gekennzeichnet, dass**

- der Sensor weiterhin einen Vorspannungskontakt (14) aufweist,
- die Vorrichtung selbstkalibrierbar ist und einen Schalter (2) aufweist, der an eine Spannungsquelle angeschlossen ist und eine nicht verformbare Basis (21) aufweist, wobei mindestens eine Seite (22) des Schalters unter Vorspannung gesetzt werden kann,

wobei der Drucksensor derart oberhalb des Schalters angeordnet ist, dass sich die Membran (12) senkrecht zur Seite (22), die unter Vorspannung gesetzt werden kann, befindet.

2. Vorrichtung nach Anspruch 1, **dadurch gekennzeichnet, dass** der Sensor (1) durch eine steife Verbindung (3) oberhalb des Schalters (2) gehalten wird, wobei der Raum zwischen der Membran (12) und der Seite (22), die unter Vorspannung gesetzt werden kann, einen Hohlraum (4) mit einer bestimmten Abmessung bildet.

3. Vorrichtung nach Anspruch 1 oder 2, **dadurch gekennzeichnet, dass** der Schalter (2) Isolationsmittel umfasst, welche die Seite (22), die unter Vorspannung gesetzt werden kann, und den Prüfkörper (11) trennen.

4. Vorrichtung nach einem der vorhergehenden Ansprüche, **dadurch gekennzeichnet, dass** der Schalter (2), auf einem isolierenden, nicht verformbaren Substrat (23), eine metallische Elektrode (24) aufweist, die an eine Spannungsquelle angeschlossen ist.

5. Vorrichtung nach Anspruch 4, **dadurch gekennzeichnet, dass** das isolierende Substrat (23) aus einem Werkstoff besteht, der aus Glas, Borosilikatglas, Glaskeramik, isolierendem Silizium gewählt ist.

6. Vorrichtung nach einem der Ansprüche 1 bis 3, **dadurch gekennzeichnet, dass** der Schalter (2) ein leitfähiges, nicht verformbares Substrat (25) aufweist, das an eine Spannungsquelle angeschlossen ist und durch mindestens ein isolierendes Element (26) vom Prüfkörper getrennt ist.

**7.** Vorrichtung nach dem vorhergehenden Anspruch, **dadurch gekennzeichnet, dass** es sich bei dem leitfähigen Substrat (25) um leitfähiges Silizium handelt und das mindestens eine isolierende Element (26) aus Siliziumoxid besteht.

**8.** Vorrichtung nach einem beliebigen der vorhergehenden Ansprüche, **dadurch gekennzeichnet, dass** der Prüfkörper (11) und die Membran (12) des Drucksensors aus leitfähigem Silizium bestehen.

**9.** Vorrichtung nach einem der vorhergehenden Ansprüche, **dadurch gekennzeichnet, dass** der Drucksensor mindestens zwei, und vorzugsweise vier, piezoelektrische Wandler (13) umfasst, die in den Bereichen der größten Verformung der Membran (12) angeordnet sind.

**10.** Vorrichtung nach einem der vorhergehenden Ansprüche, **dadurch gekennzeichnet, dass** der mindestens eine piezoelektrische Wandler aus den piezoresistiven Messvorrichtungen, den Dioden mit P-N-Übergang, den Bipolartransistoren oder den Feldeffekttransistoren gewählt ist.

**11.** Vorrichtung nach einem der vorhergehenden Ansprüche, **dadurch gekennzeichnet, dass** die piezoresistiven Messvorrichtungen aus dotiertem Silizium bestehen, welches in die Membran aus Silizium integriert ist.

**12.** Vorrichtung nach einem der vorhergehenden Ansprüche, **dadurch gekennzeichnet, dass** der Hohlraum (4) sich in einem Kanal (6) fortsetzt, der zur Atmosphäre hin offen ist.

**13.** Vorrichtung nach einem der Ansprüche 1 bis 12, **dadurch gekennzeichnet, dass** der Hohlraum (4) hermetisch geschlossen und luftleer ist.

**14.** Vorrichtung nach einem beliebigen der vorhergehenden Ansprüche, **dadurch gekennzeichnet, dass** sie darüber hinaus einen Temperatursensor umfasst.

**15.** Vorrichtung nach einem beliebigen der vorhergehenden Ansprüche, **dadurch gekennzeichnet, dass** sie an eine Zentraleinheit angeschlossen ist, die Mittel zur Verarbeitung des elektrischen Signals umfasst, welches von dem Drucksensor (1), und möglicherweise von dem Temperatursensor, erzeugt wird.

**16.** Vorrichtung nach einem beliebigen der vorhergehenden Ansprüche, **dadurch gekennzeichnet, dass** sie am Ende eines Katheters befestigt ist und von einer Hülle aus Kunstharz umgeben ist, die auf Höhe der Membran (12) ein Fenster aufweist.

**17.** Katheter zur medizinischen Verwendung, **dadurch gekennzeichnet, dass** das Ende, welches dazu bestimmt ist, im Körper eines Patienten implantiert zu werden, eine selbstkalibrierbare Vorrichtung zur Messung des Körperinnendrucks nach einem der vorhergehenden Ansprüche aufweist.

Fig1

Fig2

Fig3

## RÉFÉRENCES CITÉES DANS LA DESCRIPTION

**Documents brevets cités dans la description**

- US 2006107749 A